(19) Europäisches Patentamt
European Patent Office
Office européen des brevets

(11) Veröffentlichungsnummer: **0 166 958**
**B1**

(12) # EUROPÄISCHE PATENTSCHRIFT

(45) Veröffentlichungstag der Patentschrift: **13.01.88**

(51) Int. Cl.⁴: **C 07 C 43/11**, C 08 G 65/28

(21) Anmeldenummer: **85106494.9**

(22) Anmeldetag: **25.05.85**

(54) **Modifizierte Polyethylenglykole.**

(30) Priorität: **02.06.84 DE 3420708**

(43) Veröffentlichungstag der Anmeldung: **08.01.86 Patentblatt 86/2**

(45) Bekanntmachung des Hinweises auf die Patenterteilung: **13.01.88 Patentblatt 88/2**

(84) Benannte Vertragsstaaten: **BE CH DE FR GB IT LI NL**

(56) Entgegenhaltungen:
**EP-A-0 000 704**
**EP-A-0 055 433**
**EP-A-0 062 807**

**JOURNAL OF THE CHEMICAL SOCIETY, CHEMICAL COMMUNICATIONS, Nr. 12, 14. Juni 1978, Seiten 504-505; T.C. CLARKE et al.: "Highly conducting transition metal derivatives of polyacetylene"**

(73) Patentinhaber: **HOECHST AKTIENGESELLSCHAFT, Postfach 80 03 20, D-6230 Frankfurt am Main 80 (DE)**

(72) Erfinder: **Geymayer, Peter, Dr., Ludwigshafener Strasse 2, D-8269 Burgkirchen (DE)**
Erfinder: **Leidl, Erich, Kampenwandstrasse 24, D-8269 Burgkirchen (DE)**
Erfinder: **Wimmer, Ignaz, Burger Strasse 26, D-8261 Winhöring (DE)**
Erfinder: **Zellhuber, Ludwig, Rupertistrasse 3, D-8261 Tyrlaching (DE)**

**EP 0 166 958 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1988

**Beschreibung**

Die Erfindung betrifft modifizierte Polyethylenglykole, die sich durch einen niedrigen Erstarrungspunkt auszeichnen und bezüglich der weiteren Eigenschaften den üblichen Polyethylenglykolen gleich sind.

Polyethylenglykole sind bekanntlich Additionsprodukte von Ethylenoxid mit je einer Hydroxylgruppe am Ende der Ethylenoxid-Kette. Sie entsprechen der allgemeinen Formel $HO(CH_2CH_2O)_nH$ und stellen je nach Anzahl n der Ethylenoxid-Einheiten in der Ethylenoxid-Kette (Polymerisationsgrad) viskos-flüssige bis feste Substanzen dar. Ihre Herstellung erfolgt im allgemeinen durch Zugabe von gasförmigem oder flüssigem Ethylenoxid zu Wasser, Ethylenglykol oder Diethylenglykol in Gegenwart von Ethoxylierungskatalysatoren.

Aufgrund ihrer Eigenschaften können Polyethylenglykole vielfach angewendet werden, beispielsweise in der Kosmetik, in der Textil- und Lederindustrie, in der Gummi- und Elastomerindustrie, auf dem Schmiermittelsektor, in der Metallbearbeitung und Holzbehandlung, zur Klebstoff-Herstellung und als Wärmeübertragungsmittel. Zu den vorteilhaften Eigenschaften zählen insbesondere die Wasserlöslichkeit, die biologische Abbaubarkeit, die Unflüchtigkeit und die hohe Hitzebeständigkeit sowie die weitgehende gesundheitliche Unbedenklichkeit.

Neben den vielen wertvollen Eigenschaften weisen die bekannten Polyethylenglykole aber auch eine sehr nachteilige Eigenschäft auf, nämlich einen relativ hohen Erstarrungspunkt, woraus insbesondere Schwierigkeiten bezüglich ihrer Handhabung resultieren. So liegt die Erstarrungstemperatur von Polyethylenglykol mit einem mittleren Molekulargewicht (mittleren molaren Masse) von etwa 200 um -50° C. Von Polyethylenglykol mit einem mittleren Molekulargewicht von etwa 300 um -12° C, von Polyethylenglykol mit einem mittleren Molekulargewicht von etwa 400 bis 600 um +4 bis +20° C und von einem Polyethylenglykol mit einem mittleren Molekulargewicht von 700 bis 1500 um 25 bis 45° C. Diese hohen Erstarrungspunkte machen zur Erzielung des flüssigen Zustandes eine Lagerung der Produkte bei höheren Temperaturen erforderlich oder ein Aufheizen vor ihrer Verwendung, was viel Zeit und Energie kostet. Öfteres Erhitzen kann zudem die Farbqualität der Produkte aufgrund von Oxidationsreaktionen verschlechtern. Bei vielen Anwendungen, beispielsweise bei der Verwendung als Schmiermittel, als Metallbearbeitungsmittel und dergleichen, ist mit dem Aufheizen noch immer kein störungsfreier Betrieb gewährleistet, da es während des Betriebes unter Umständen zu einer Wiedererstarrung kommen kann. Aus den bekannten Polyethylenglykolen resultieren ferner Umsetzungsprodukte, die ihrerseits relativ hohe Erstarrungspunkte besitzen.

Außer den reinen Polyethylenglykolen sind auch Polyethylen-Polypropylenglykole bekannt. Dazu gehören jene mit statistischer Verteilung der Ethylenoxid- und Propylenoxid-Einheiten im Kettenmolekül und ferner die Blockpolymerisate aus Ethylenoxid und Propylenoxid. Zu den Blockpolymerisaten zählen solche der allgemeinen Formel

$$HO(CH_2CH_2O)_a(CH_2\overset{\overset{\displaystyle CH_3}{|}}{C}HO)_b(CH_2CH_2O)_cH,$$

worin a, b und c für die Anzahl der Ethylenoxid-Gruppen und Propylenoxid-Gruppen stehen. Sie enthalten eine beträchtliche Menge an Propylenoxid und unterscheiden sich von den reinen Polyethylenglykolen unter anderem auch dadurch, daß sie im Gegensatz zu diesen oberflächenaktiv und nicht vollständig biologisch abbaubar sind (vergleiche US-PS-2 674 619 und DE-OS-18 15 362).

In Journal of the Chemical Society, Chemical Communications, Nr. 12/1978, Seiten 504 und 505, ist ein Verfahren zur Herstellung von Kronenethern beschrieben, bei dem ein Polyethylenglykol mit einem Sulfonylchlorid in Gegenwart von Alkalimetallhydroxiden zu dem angestrebten Kronenether umgesetzt wird. Dabei wird als Ausgangsglykol unter anderen auch das Polyethylenglykol der Formel

$$HO(CH_2CH_2O)_m(CH_2CHR_1O)\ (CH_2CH_2I)_nH$$

genannt, worin $R_1 = CH_3$ und $m + n = 5$ ist. Die Eigenschaften der eingesetzten Polyethylenglykole werden nicht abgehandelt.

Schließlich werden in der EP-A1-0 062 807 mit Strahlen härtbare Acrylsäureester beschrieben, die durch Reaktion von Acrylsäure mit einem Diol hergestellt werden, wobei das Diol ausgewählt ist aus einem ethoxylierten und/oder propoxylierten 1,6-Hexandiol, Neopentylglykol oder einem Tripropylenglykol mit jeweils relativ niedrigem Alkoxylierungsgrad. Dabei wird als Diol auch die Verbindung genannt, die sich ergibt, wenn an einem mol Neopentylglykol 2 bis 6 mol Ethylenoxid angelagert werden. Über die Eigenschaften der eingesetzten Diole wird nichts weiteres gesagt.

Die Aufgabe der Erfindung besteht nun darin, derart modifizierte Polyethylenglykole zur Verfügung zu stellen, die im Vergleich zu den üblichen oder reinen Polyethylenglyglykolen einen wesentlich tieferen Erstarrungspunkt aufweisen und ansonsten die gleichen Eigenschaften wie diese besitzen.

Die erfindungsgemäßen modifizierten Polyethylenglykole sind dadurch gekennzeichnet, daß sie der nachstehenden allgemeinen Formel I entsprechen:

$$HO(CH_2CH_2O)_x(RO)_y(CH_2CH_2O)_zH, \quad (I)$$

worin R für einen einfach- oder mehrfach-, vorzugsweise ein- oder zweifach-alkylsubstituierten Alkylenrest mit 2 bis 4 Kohlenstoffatomen in der Alkylenkette und mit Methyl, Ethyl, Propyl oder Isopropyl als Alkylsubstituent steht, y 1 bis 2, vorzugsweise 1, und die Summe von x und z 5 bis 30, vorzugsweise 10 bis 20, beträgt, wobei weder x noch z Null ist, mit der Maßgabe, daß die Verbindung mit R = $-CH_2CH(CH_3)-$, y = 1 und x + z = 5 und die Verbindungen mit R = $-CH_2C(CH_3)_2CH_2-$, y = 1 und x + z = 5 oder 6 ausgenommen sind.

R ist erfindungsgemäß eine bestimme verzweigte Alkylengruppe. Sie besteht aus 2 bis 4, vorzugsweise 2 oder 3, Kohlenstoffatomen in der Alkylenkette und trägt als Verzweigungen $C_1$- bis $C_3$-Alkylgruppen, vorzugsweise die Methylgruppe. Die Verzweigungen (die Alkylsubstituenten), die gleich oder verschieden sind, können einmal oder mehrmals vorhanden sein, vorzugsweise ist die Alkylenkette ein- bis zweimal substituiert.

Bevorzugte Polyethylenglykole gemäß Formel I sind auch solche, wenn der Alkylenrest ein- oder zweifach alkylsubstituiert ist, y 1 und die Summe von x und z 10 bis 20 beträgt.

Bevorzugte Vertreter von R, das heißt, bevorzugte $C_1$-bis $C_3$-alkylsubstituierte Alkylenreste sind:

| Formel | Bezeichnung |
|---|---|
| $CH_3$<br>\|<br>$-CH_2CH-$ | 1-Methylethylenrest<br>(Isopropylenrest) |
| $CH_3$<br>\|<br>$-CH_2CH_2CH-$ | 1-Methylpropylenrest |
| $CH_3$<br>\|<br>$-CH_2CHCH_2-$ | 2-Methylpropylenrest |
| $CH_3$<br>\|<br>$-CH_2CHCH_2CH_2-$ | 2-Methylbutylenrest |
| $CH_3$<br>\|<br>$-CH_2CCH_2-$<br>\|<br>$CH_3$ | 2.2-Dimethylpropylenrest |
| $CH_3$<br>\|<br>$-CH_2CCH_2CH_2-$<br>\|<br>$CH_3$ | 2.2-Dimethylbutylenrest |
| | FIG1/10 |
| $CH_3$ $CH_3$<br>\|<br>$-CH_2CCHCH_2-$<br>\|<br>$CH_3$ | 2.2.3-Trimethylbutylenrest |

wovon der Isopropylenrest und der 2.2-Dimethylpropylenrest besonders bevorzugt sind.

Zur weiteren Veranschaulichung werden nachstehend die den oben angeführten Alkylenresten entsprechenden Glykole (Diole oder Dihydroxy-Verbindungen) angegeben, von denen der RO-Rest aus der Formel I stammt und von denen bei der Herstellung der erfindungsgemäßen Polyethylenglykole ausgegangen wird:

| Formel | Bezeichnung |
|---|---|
| $CH_3$<br>\|<br>$HOCH_2CHOH$ | 1-Methylethylenglykol<br>(1.2-Propylenglykol) |
| $CH_3$<br>\|<br>$HOCH_2CH_2CHOH$ | 1-Methylpropylenglykol<br>(Butandiol-1,3) |
| $CH_3$<br>\|<br>$HOCH_2CHCH_2OH$ | 2-Methylpropylenglykol |
| $CH_3$<br>\|<br>$HOCH_2CHCH_2CH_2OH$ | 2-Methylbutylenglykol |
| $CH_3$<br>\|<br>$HOCH_2CCH_2OH$<br>\|<br>$CH_3$ | 2.2-Dimethylpropylenglykol<br>(Neopentylglykol) |
| $CH_3$<br>\|<br>$HOCH_2CCH_2CH_2OH$<br>\|<br>$CH_3$ | 2.2-Dimethylbutylenglykol |
| $CH_3$ $CH_3$<br>\|<br>$HOCH_2CCHCH_2OH$<br>\|<br>$CH_3$ | FIG2/10<br>2.2.3-Trimethylbutylenglykol |

Besonders bevorzugte modifizierte Polyethylenglykole sind demnach solche der nachstehenden Formeln II und III

$$HO(CH_2CH_2O)_x(CH_2\overset{\overset{\displaystyle CH_3}{|}}{C}HO)_y(CH_2CH_2O)_zH \qquad (II)$$

$$HO(CH_2CH_2O)_x(CH_2\text{-}\underset{\underset{\displaystyle CH_3}{|}}{\overset{\overset{\displaystyle CH_3}{|}}{C}}\text{-}CH_2O)_y(CH_2CH_2O)_zH, \qquad (III)$$

worin y 1 bis 2, vorzugsweise 1, beträgt, und die Summe von x und z 5 bis 30, vorzugsweise 10 bis 20 beträgt, wobei weder x noch z Null ist und wobei die oben angegebene Maßgabe gilt.

Die Herstellung der erfindungsgemäßen modifizierten Polyethylenglykole erfolgt im Prinzip nach den für die Anlagerung von Ethylenoxid an Glykole bekannten Arbeitsweisen. Dabei wird ein dem Rest in Formel I entsprechendes Glykol, also ein ausgewähltes verzweigtes Monoalkylenglykol (y = 1) oder Dialkylenglykol (y = 2), eingesetzt und ein spezielles molares Verhältnis zwischen dem eingesetzten Glykol und dem anzulagernden Ethylenoxid eingehalten, und zwar ein Verhältnis von 5 bis 30 mol Ethylenoxid, vorzugsweise 10 bis 20 mol Ethylenoxid, pro mol Alkylen- oder Dialkylenglykol Die Herstellung der erfindungsgemäßen Polyethylenglykole erfolgt im einzelnen in der Regel in der Weise, daß in einem mit Rührer ausgestatteten Druckgefäß die verzweigte Glykolverbindung und ein alkalischer Katalysator vorgelegt werden, worauf zur Erzeugung einer inerten Atmosphäre vorzugsweise mit Stickstoff gespült wird. Zur Entfernung von Wasser (das beispielsweise von der Einbringung des Alkalikatalysators oder von der Bildung von Alkaliglykolat stammt) und/oder von Lösungsmitteln wie Methanol (das ebenfalls von der Einbringung des Alkalikatalysators stammen kann) wird zweckmäßigerweise unter Vakuum (Wasserstrahlvakuum) erhitzt, bis dieses ausgetragen ist. Nun

wird bei einer Temperatur von 110 bis 170°C, vorzugsweise 120 bis 160°C, flüssiges oder gasförmiges Ethylenoxid zudosiert und angelagert, wobei sich ein Druck im Bereich von 30 bis 400 kPa einstellt. Das Ende der Ethylenoxid-Addition ist am abgefallenen und im wesentlichen konstant bleibenden Druck erkennbar.

Zur Entfernung von gegebenenfalls anwesenden flüchtigen Anteilen hat es sich als zweckmäßig erwiesen, das so erhaltene erfindungsgemäße Polyethylenglykol bei einem Vakuum von etwa 2 bis 10 kPa 0,3 bis 1 h lang auf etwa 80 bis 120°C zu halten.

Geeignete alkalische Katalysatoren sind Alkalihydroxide, Alkalicarbonate und Alkalialkoholate mit 1 bis 3 Kohlenstoffatomen, wobei die entsprechenden Natrium- und Kaliumverbindungen bevorzugt sind. Besonders bevorzugte alkalische Katalysatoren sind Natriumhydroxid, Natriumcarbonat und Natriummethylat sowie die entsprechenden Kaliumverbindungen. Die Menge an Katalysator beträgt im allgemeinen 0,1 bis 5 Gew.-%, vorzugsweise 0,3 bis 3 Gew.-%, bezogen auf die Menge an eingesetztem Glykol.

Die erfindungsgemäßen modifizierten Polyethylenglykole stellen ebenso wie die bekannten reinen Polyethylenglykole (je nach Molekulargewicht) viskos-flüssige bis feste Produkte dar. Sie besitzen im Vergleich zu den reinen Polyethylenglykolen überraschenderweise einen beträchtlich niedrigeren Erstarrungspunkt, stimmen aber mit diesen in allen anderen wichtigen Eigenschaften wunschgemäß überein. Aufgrund dieser unerwarteteten Erscheinung sind die neuen Polyethylenglykole bezüglich Handhabung und Lagerung klarerweise viel vorteilhafter und bei allen Anwendungen, bei denen der flüssige Zustand innerhalb eines relativ weiten Temperaturbereiches erhalten bleiben soll, viel unproblematischer als die üblichen Polyethylenglykole. Es hat sich ferner gezeigt, daß auch die Umsetzungsprodukte der neuen Polyethylenglykole, beispielsweise die Veretherungsprodukte, vergleichsweise niedrigere Erstarrungspunkte aufweisen, was einen weiteren großen Vorteil darstellt.

Die Erfindung wird nun an Beispielen noch näher erläutert.

**Beispiel 1**

In einem mit Rührer ausgestatteten Reaktionsgefäß wurden 104 g (1 mol) Neopentylglykol und 2 g einer 30 gew.-%-igen methanolischen Natriummethylatlösung (0,6 Gew.-% Natriummethylat, bezogen auf Neopentylglykol) vorgelegt. Nach Spülen mit Stickstoff zur Entfernung des Sauerstoffs wurde auf 120 bis 125°C erhitzt und bei dieser Temperatur unter Rühren 2 h lang unter Wasserstrahlvakuum zur Entfernung des vorhandenen Methanols gehalten.

Nach Wegnahme des Wasserstrahlvakuums wurde unter Rühren auf 130 bis 140°C erhitzt, worauf bei dieser Temperatur in einer Zeit von etwa 3 h 502 g (11,4 mol) gasförmiges Ethylenoxid zudosiert wurde, wobei ein Druck im Bereich von 100 bis 400 kPa vorlag (das Ende der Ethylenoxid-Addition war am abgefallenen und im wesentlichen konstant bleibenden Druck erkennbar). Zur Reinigung des Reaktionsproduktes von gegebenenfalls anwesenden flüchtigen Anteilen wurde dieses eine halbe Stunde lang unter Rühren bei etwa 80°C und einem Vakuum von etwa 2 kPa gehalten.

**Beispiele 2 bis 8**

Die Beispiele 2 bis 8 wurden analog dem Beispiel 1 durchgeführt. Die vorgelegten Glykole und alkalischen Katalysatoren sowie die bei Reaktionstemperatur und dem Reaktionsdruck zudosierte Ethylenoxid-Menge sind in der nachstehenden Tabelle 1 zusammengefaßt. Die Tabelle enthält aus Gründen der Übersicht auch die entsprechenden werte des Beispiels 1.

In der nachstehenden Tabelle 2 sind in Anlehnung an Formel I bzw. an die Formeln II und III die Werte für y und x + z der Produkte gemäß den Beispielen 1 bis 8 angegeben und daneben ihre Erstarrungspunkte und als weitere Eigenschaften ihre mittlere molare Masse (Molmasse), Viskosität bei 25°C und ihre Löslichkeit in Wasser (die für x, y und z angegebenen Zahlen stellen statistische Mittelwerte dar).

Um einen Vergleich zwischen den erfindungsgemäßen modifizierten Polyethylenglykolen und den bekannten (reinen) Polyethylenglykolen mit der gleichen mittleren molaren Masse zu haben, sind in Tabelle 2 bei "Erstarrungspunkt" auch die entsprechenden Werte der bekannten Polyethylenglykole angeführt (in den übrigen Eigenschaften haben beide Polyethylenglykole im wesentlichen die gleichen Werte).

Wie die Beispiele zeigen, zeichnen sich die neuen modifizierten Polyethylenglykole im Vergleich zu den bekannten durch einen unerwartet niedrigen Erstarrungspunkt aus. Dazu kommt noch ein weiterer großer Vorteil, nämlich der, daß die erfindungsgemäßen Polyethylenglykole in anderen wichtigen Eigenschaften den bekannten Polyethylenglykolen im wesentlichen gleich sind.

**Tabelle I**

| Beispiel Nr. | Glykol | | Ethylenoxid | | Katalysator | | Reaktionstemperatur | Reaktions-Druck |
|---|---|---|---|---|---|---|---|---|
| | (g) | (mol) | (g) | (mol) | Art | (Gew.-%) | (°C) | (kPa) |
| 1 | Neopentylglykol 104,0 | 1,0 | 502,0 | 11,4 | $CH_3ONa$ | 0,6 | 130 - 140 | 100 - 400 |
| 2 | Neopentylglykol 104,0 | 1,0 | 871,2 | 19,8 | $CH_3ONa$ | 0,8 | 135 - 140 | 50 - 400 |
| 3 | 1,2-Propylenglykol 76,0 | 1,0 | 510,4 | 11,6 | NaOH | 1,0 | 140 - 150 | 50 - 400 |
| 4 | 1,2-Propylenglykol 76,0 | 1,0 | 660,0 | 15,0 | $Na_2CO_3$ | 3,0 | 140 - 150 | 30 - 300 |
| 5 | 1,2-Propylenglykol 76,0 | 1,0 | 880,0 | 20,0 | $K_2CO_3$ | 2,5 | 140 - 150 | 50 - 400 |
| 6 | 1,2-Propylenglykol 76,0 | 1,0 | 1276,0 | 29,0 | NaOH | 2,0 | 120 - 140 | 50 - 400 |
| 7 | 1,3-Butandiol 90,1 | 1,0 | 510,4 | 11,6 | KOH | 1,0 | 140 - 150 | 30 - 300 |
| 8 | Dipropylenglykol 134,0 | 1,0 | 466,0 | 10,6 | KOH | 0,7 | 140 - 150 | 30 - 300 |

**Tabelle II**

| Beispiel Nr. | y | x + z | Mittlere molare Masse (g/mol) | Erstarrungspunkt nach DIN 51 570 v.modifiziertem PEG*) (°C) | v. üblichem PEG*) gleicher Molmasse (°C) | Viskosität bei 25°C nach DIN 51 562 (mPa s) | Wasserlöslichkeit |
|---|---|---|---|---|---|---|---|
| 1 | 1 | 11,4 | 606 | -4 | +20 | 125 | klar löslich |
| 2 | 1 | 19,8 | 1020 | +14 | +37 | 285 | klar löslich |
| 3 | 1 | 11,6 | 600 | -5 | +20 | 120 | klar löslich |
| 4 | 1 | 15,0 | 750 | +4 | +26 | 150 | klar löslich |
| 5 | 1 | 20,0 | 1000 | +15 | +37 | 220 | klar löslich |
| 6 | 1 | 29,0 | 1350 | +20 | +43 | fest | klar löslich |
| 7 | 1 | 11,6 | 617 | -3 | +20 | 133 | klar löslich |
| 8 | 2 | 10,6 | 600 | -27 | +20 | 110 | klar löslich |

*) PEG = Polyethylenglykol

**Patentansprüche**

1. Modifizierte Polyethylenglykole der allgemeinen Formel I

$$HO(CH_2CH_2O)_x(RO)_y(CH_2CH_2O)_zH, \quad (I)$$

worin R für einen einfach oder mehrfach alkylsubstituierten Alkylenrest mit 2 bis 4 Kohlenstoffatomen in der Alkylenkette und mit Methyl, Ethyl, Propyl oder Isopropyl als Alkylsubstituent steht, y 1 bis 2 und die Summe von x und z 5 bis 30 beträgt, wobei weder x noch z Null ist, mit der Maßgabe, daß die Verbindung mit R = $-CH_2CH(CH_3)-$, y = 1 und x + z = 5 und die Verbindungen mit R = $-CH_2C(CH_3)_2CH_2-$, y = 1 und x + z = 5 oder 6 ausgenommen sind.

2. Polyethylenglykole nach Anspruch 1, dadurch gekennzeichnet, daß der Alkylenrest ein- oder zweifach alkylsubstituiert ist.

3. Polyethylenglykole nach Anspruch 1, dadurch gekennzeichnet, daß y 1 ist.

4. Polyethylenglykole nach Anspruch 1, dadurch gekennzeichnet, daß die Summe von x und z 10 bis 20 beträgt.

5. Polyethylenglykole nach Anspruch 1, dadurch gekennzeichnet, daß der Alkylenrest ein- oder zweifach alkylsubstituiert ist, y 1 und die Summe von x und z 10 bis 20 beträgt.

6. Polyethylenglykole nach Anspruch 1, dadurch gekennzeichnet, daß sie einer der beiden nachstehenden allgemeinen Formeln II oder III entsprechen:

$$\begin{array}{c} CH_3 \\ | \\ HO(CH_2CH_2O)_x(CH_2CHO)_y(CH_2CH_2O)_zH \end{array} \qquad (II)$$

$$\begin{array}{c} CH_3 \\ | \\ HO(CH_2CH_2O)_x(CH_2\text{-}C\text{-}CH_2O)_y(CH_2CH_2O)_zH, \\ | \\ CH_3 \end{array} \qquad (III)$$

worin y 1 und die Summe von x und z 10 bis 20 beträgt.

**Claims**

1. Modified polyethylene glycols of the general formula

$HO(CH_2CH_2O)_x(RO)_y(CH_2CH_2O)_zH$, (I)

in which R is a single or multiple alkyl-substituted alkylene radical having 2 to 4 carbon atoms in the alkylene chain and having methyl, ethyl, propyl or isopropyl as the alkyl-substituent, y is 1 to 2 and the sum of x and z is 5 to 30, neither x nor z being zero, with the proviso that the compound having R = $-CH_2CH(CH_3)-$, y = 1 and x + z = 5, and the compounds having R = $-CH_2C(CH_3)_2CH_2-$, y = 1 and x + z = 5 or 6 are excluded.

2. Polyethylene glycols as claimed in claim 1, wherein the alkylene radical is single or twice alkyl-substituted.

3. Polyethylene glycols as claimed in claim 1, wherein y is 1.

4. Polyethylene glycols as claimed in claim 1, wherein the sum of x and z is 10 to 20.

5. Polyethylene glycols as claimed in claim 1, wherein the alkylene radical is single or twice alkyl-substituted, y is 1 and the sum of x and z is 10 to 20.

6. Polyethylene glycols as claimed in claim 1, corresponding to the following two formulae II or III:

$$\begin{array}{c} CH_3 \\ | \\ HO(CH_2CH_2O)_x(CH_2CHO)_y(CH_2CH_2O)_zH \end{array} \qquad (II)$$

$$\begin{array}{c} CH_3 \\ | \\ HO(CH_2CH_2O)_x(CH_2\text{-}C\text{-}CH_2O)_y(CH_2CH_2O)_zH, \\ | \\ CH_3 \end{array} \qquad (III)$$

wherein y is 1 and the sum of x and z is 10 to 20.

**Revendications**

1. Polyéthylèneglycols modifiés de formule générale I

$HO(CH_2CH_2O)_x(RO)_y(CH_2CH_2O)_zH$, (I)

dans laquelle R représente un radical alkylène ayant de 2 à 4 atomes de carbone dans la chaîne alkylène, portant un ou plusieurs substituants alkyle, les substituants alkyle étant les radicaux méthyle, éthyle, propyle ou isopropyle, y vaut 1 à 2, et la somme de x et de z vaut de 5 à 30, ni x ni z n'étant égaux à zero, à la condition que soient exclus le composé dans lequel R = $-CH_2CH(CH_3)-$, y = 1 et x + z = 5, et les composés dans lesquels R = $-CH_2C(CH_3)_2CH_2-$, y = 1 et x + z = 5 ou 6.

2. Polyéthylèneglycols selon la revendication 1, caractérisés en ce que le radical alkylène porte un ou deux substituants alkyle.

3. Polyéthylèneglycols selon la revendication 1, caractérisés en ce que y = 1.

4. Polyéthylèneglycols selon la revendication 1, caractérisés en ce que la somme de x et de z vaut de 10 à 20.

5. Polyéthylèneglycols selon la revendication 1, caractérisés en ce que le radical alkylène porte un ou deux substituants alkyle, y vaut 1, et la somme de x et de z vaut de 10 à 20.

6. Polyéthylèneglycols selon la revendication 1, caractérisés en ce qu'ils correspondent à l'une des formules générales II ou III suivantes:

$$\begin{array}{l} \qquad\qquad\qquad\quad CH_3 \\ \qquad\qquad\qquad\quad | \\ HO(CH_2CH_2O)_x(CH_2CHO)_y(CH_2CH_2O)_zH \end{array} \qquad (II)$$

$$\begin{array}{l} \qquad\qquad\qquad\qquad CH_3 \\ \qquad\qquad\qquad\qquad | \\ HO(CH_2CH_2O)_x(CH_2\text{-}C\text{-}CH_2O)_y(CH_2CH_2O)_zH, \\ \qquad\qquad\qquad\qquad | \\ \qquad\qquad\qquad\qquad CH_3 \end{array} \qquad (III)$$

dans lesquelles y vaut 1, et la somme de x et de z vaut de 10 à 20.